(19) Europäisches Patentamt
European Patent Office
Office européen des brevets

(11) **EP 1 238 057 B1**

(12) **FASCICULE DE BREVET EUROPEEN**

(45) Date de publication et mention
de la délivrance du brevet:
**21.12.2005 Bulletin 2005/51**

(21) Numéro de dépôt: **00985397.9**

(22) Date de dépôt: **04.12.2000**

(51) Int Cl.⁷: **C12N 5/02**, C12N 5/06,
C12N 7/00

(86) Numéro de dépôt international:
**PCT/FR2000/003377**

(87) Numéro de publication internationale:
**WO 2001/040443 (07.06.2001 Gazette 2001/23)**

(54) **METHODE DE PRODUCTION DE CELLULES ANIMALES ADHERENTES**

VERFAHREN ZUR ZÜCHTUNG VON HAFTENDEN TIERISCHEN ZELLEN

METHOD FOR PRODUCING ADHERENT ANIMAL CELLS

(84) Etats contractants désignés:
**AT BE CH CY DE DK ES FI FR GB GR IE IT LI LU
MC NL PT SE TR**

(30) Priorité: **03.12.1999 FR 9915303**

(43) Date de publication de la demande:
**11.09.2002 Bulletin 2002/37**

(73) Titulaire: **Sanofi Pasteur
69367 Lyon Cedex 07 (FR)**

(72) Inventeur: **GERDIL, Catherine
F-69160 Tassin (FR)**

(74) Mandataire: **Ayroles, Marie-Pauline et al
2, Avenue Pont Pasteur
69367 Lyon Cedex 07 (FR)**

(56) Documents cités:
**EP-A- 0 354 129         WO-A-88/00967
WO-A-98/24883**

**Description**

La présente invention se rapporte à une méthode de production de cellules animales adhérentes dans un milieu de culture dépourvu de sérum d'origine animale en vue de la fabrication de vaccins viraux ainsi qu'à l'utilisation d'une molécule de polyvinylpyrrolidone (PVP).

État de la technique

Les milieux de culture sont généralement constitués d'un milieu de base, qui contient les éléments nutritifs essentiels au développement des cellules, complémenté par du sérum sanguin, le plus souvent du sérum de veau du donneur ou du sérum de veau foetal.

Des milieux de culture dépourvus en sérum d'origine animale ont été rapportés pour induire la prolifération de cellules non adhérentes en suspension, notamment la culture de lignées d'hybridomes (Kovar and Franek, Biotechnol. Lett. 9:259-264 (1987); Murakami H., in Growth of cells hormonally defined media (G. Sato et al., eds), Cold Spring Harbour Laboratories, Cold Spring Harbour, New York, p. 711-715 (1982); Brevet JP 63084487).

On sait que les milieux de culture dépourvus en sérum d'origine animale sont inadaptés pour la culture de cellules adhérentes (Kovar J., In vitro Cell. Dev. Biol. 25:395-396 (1989)). L'attachement des cellules adhérentes à un support nécessite des facteurs d'attachement apportés par le milieu de culture tels que la fibronectine, la vitronectine, la laminine, le collagène (Koller M. R. and Papoutsakis E. T., Bioprocess. Technol. 20:61-110 (1995). Ces facteurs d'attachement sont présents essentiellement dans le sérum d'origine animale (Hayman E., et al., Exp. Cell. Res. vol. 160: 245 (1985)).

La demande WO 88/00967 décrit un procédé de culture de cellules de mammifères adhérentes dans un biogénérateur comprenant une étape de culture dans un milieu contenant du sérum pour favoriser l'attachement des cellules à un support et permettre la prolifération des cellules jusqu'à une concentration désirée, suivie d'une étape dans laquelle le milieu de culture est éliminé puis remplacé par un milieu dépourvu de sérum, contenant par ailleurs une polyvinylpyrrolidone, du polyéthylène glycol ou un polymère constitué d'un ou plusieurs groupes oxyde pour protéger les cellules adhérentes en réduisant le drainage filmogène autour des cellules. Ce procédé sert à la production de molécules recombinantes par les cellules. Il n'est pas mentionné qu'une PVP agit sur la prolifération cellulaire.

La demande FR 2 635 008 décrit un milieu de culture défini, dépourvu de sérum, contenant par ailleurs une PVP ayant un poids moléculaire de 40KD (PVP 40) à une concentration d'au moins 3% dans le milieu, pour favoriser la production d'interleukine 2 dans le surnageant de culture par une lignée CHO adhérente recombinante cultivée sur des microporteurs placés dans un réacteur soumis à une agitation mécanique. Il est aussi clairement indiqué qu'un milieu de culture avec une PVP 40 n'est pas meilleur que le même milieu sans PVP 40 pour assurer la croissance de cette lignée (page 7, lignes 15 à 25; page 8, lignes 5 à 8).

La demande WO 98/24883 décrit la composition d'un milieu de culture dépourvu de sérum, contenant un composé stéroïdien et accessoirement du PLURONIC F68 comme agent surfactant et une PVP de 10KD (PVP 10) comme agent détoxifiant, pour favoriser la croissance de lignées adhérentes, comme la lignée Vero placée dans un environnement statique, c'est à dire un environnement dans lequel la lignée n'est pas soumise au flux d'agitation du milieu de culture. La présence de stérols dans le milieu de culture est indispensable pour observer une croissance cellulaire.

Selon l'enseignement de l'art antérieur, on peut raisonnablement douter qu'un milieu sans sérum contenant une PVP puisse assurer la multiplication de lignées adhérentes lorsqu'elles sont placées dans un milieu de culture régulièrement agité, un environnement souvent rencontré lors de la production de cellules en grande quantité ou à grande échelle. En effet, la demande FR 2 635 008 montre qu'une PVP utilisée comme substitut du sérum n'augmente pas la multiplication de cellules adhérentes à des microporteurs soumis à une agitation ambiante régulière. L'article de Gyun M. et al. (Hybridoma, Vol 8: 639-645), précise par ailleurs que le sérum est indispensable lorsque le milieu de culture est régulièrement agité (page 639).

Il existe un besoin dans le domaine de la culture de cellules de trouver un substitut au sérum d'origine animale car son utilisation est très contraignante. En tant que produit d'origine biologique, le sérum fait l'objet de contraintes réglementaires très strictes d'utilisation pour éviter la transmission de virus animaux à l'homme. De plus, les composants du sérum ne sont pas tous très bien identifiés et sont en quantité variable d'un lot à l'autre, d'où la nécessité de contrôler l'activité biologique de ces lots et plus particulièrement leur action sur la croissance cellulaire de façon à ne sélectionner que les meilleurs. La présence d'une grande quantité de protéines dans le sérum peut aussi compliquer singulièrement les méthodes d'isolement et de purification des composants cellulaires et dérivés des cellules comme les virus.

Il existe également un besoin de trouver un milieu de culture dépourvu de sérum qui favorise la croissance de cellules dans un milieu régulièrement agité, un environnement généralement rencontré lors de la production de cellules en grande quantité. L'agitation mécanique ou médiée par un flux gazeux est en effet une condition nécessaire car elle favorise l'apport de nutriments mais elle a l'inconvénient de fragiliser les cellules à cause des forces de cisaillement qu'elle engendre.

L'objet de la présente invention pallie ces besoins en proposant une nouvelle méthode de production d'une classe de cellules animales ou humaines particulières.

Résumé de l'invention

A cet effet, la présente invention concerne tout d'abord un procédé de production, dans un milieu de culture dépourvu en sérum d'origine animale, de cellules animales ou humaines non recombinantes adhérentes comprenant:

(i) une première étape dans laquelle on ensemence dans un dispositif de culture comprenant un support d'adhésion, un milieu de culture dépourvu en sérum d'origine animale et contenant une polyvinylpyrrolidone d'un poids moléculaire moyen compris entre 20 KD et 360 KD avec une suspension de cellules animales ou humaines non recombinantes;
(ii) une deuxième étape dans laquelle les cellules se multiplient sous agitation dans le même milieu de culture; et
(iii) une troisième étape dans laquelle on récolte les cellules lorsqu'elles ont atteint un pallier de croissance.

Elle concerne également un procédé dans lequel le milieu de culture a une composition chimiquement définie.
Selon un mode de réalisation du procédé, la polyvinylpyrrolidone a un poids moléculaire moyen de 40 kDa.
Selon un autre mode de réalisation, le pourcentage de polyvinylpyrrolidone dans le milieu de culture est compris entre 0,01% et 2%.
De façon particulière, le pourcentage de polyvinylpyrrofidone dans le milieu de culture est de 0,1%.
L'invention concerne également un procédé dans lequel le support d'adhésion est constitué par des microporteurs.
De façon préférée, les microporteurs sont constitués par une matrice de dextran substituée par des goupements N, N diethylaminoéthyle.
L'invention a également pour objet un procédé dans lequel le dispositif de culture est un biogénérateur.
Dans un autre aspect du procédé de l'invention, le mode de culture est le mode batch, feed batch ou par perfusion continue.
L'invention a finalement pour objet un procédé dans lequel les cellules sont les cellules de la lignée Vero ou MRC5.
Contre toute attente, une PVP de 20KD à 360KD, et préférentiellement une PVP de 40KD dans un milieu de culture dépourvu de sérum peut assurer avec une efficacité comparable à celle d'un milieu avec sérum, la croissance de cellules adhérentes soumises à un flux d'agitation du milieu. D'une façon plus surprenante une PVP de 10 KD testée sur les mêmes cellules et utilisée dans les mêmes conditions n'a pas d'effet significatif sur la croissance alors que la demande WO 98/24883 relate un milieu de culture défini contenant une PVP de 10 KD, des composés stéroïdiens en présence de surfactants pour favoriser la croissance de lignées adhérentes placées dans un environnement statique.

Description détaillée de l'invention

Dans le contexte de la présente invention, différents termes employés sont ci-après définis:

" Par Polyvinylpyrrolidone ou PVP" on entend un enchaînement du monomère de 1-vinyl-2-pyrrolidone par liaison de covalence, de façon à obtenir un polymère du monomère. Lorsque l'enchaînement est linéaire et ne possède pas de ramification on considère que la polyvinylpyrrolidone est non réticulée.

"Par milieu de culture ayant une composition chimiquement définie" on entend un milieu comprenant un nombre défini de composants bien caractérisés sur le plan moléculaire et utilisés à des concentrations également bien déterminées. Un milieu de culture contenant du sérum d'origine animale n'est donc pas un milieu de culture chimiquement défini.

"Par milieu de culture dépourvu en sérum d'origine animale" on entend un milieu de culture ne contenant pas de sérum ou de produits extraits de sérums animaux et notamment ceux provenant de mammifères, d'oiseaux, de poissons ou de crustacés. Le milieu de culture, quant à lui, peut avoir une composition chimiquement défini ou chimiquement non défini s'il contient par exemple des extraits de micro-organismes, de levures ou de champignons ou même de végétaux qui ne sont pas bien caractérisés sur le plan chimique.

Par "cellules adhérentes" on entend des cellules établies en lignées ou des cellules issues directement de l'extraction de tissus animaux ou humains sains ou tumoraux qui ont besoin d'un support solide pour se multiplier et se développer normalement. Une caractéristique essentielle des cellules adhérentes est de former une couche unicellulaire uniforme sur leur support dû au phénomène d'inhibition de contact. On exclut donc de fait les cellules qui n'ont pas besoin de support solide pour se multiplier. En général, ces cellules poussent en suspension dans le milieu de culture, comme par exemple les lignées d'hybridomes. Les lignées adhérentes peuvent être issues de cultures primaires de cellules saines ou tumorales mais également peuvent être obtenues par transformation de cellules à l'aide d'agents immortalisants. Les lignées adhérentes utilisées peuvent être mortelles (durée de vie limitée) ou immortelles (durée de vie illimitée).

Par "flux d'agitation du milieu" on entend un milieu de culture agité mécaniquement et/ou sous l'action d'un courant gazeux.

Par "index de prolifération " on entend le rapport entre le nombre maximal de cellules obtenues par ml de milieu de culture contenant une PVP comme substitut du sérum et le nombre maximal de cellules obtenues par ml du même milieu de culture mais dépourvu de PVP. Pour le calcul de cet index, il est entendu que les cellules et les conditions de culture sont identiques. Le nombre maximal de cellules obtenues dans chacun des deux milieux testés n'est pris en compte que lorsque la courbe de croissance observée pour chacun des milieux a atteint une phase dite stationnaire, ou "palier" témoignant de l'arrêt de la prolifération des cellules. Une valeur de l'index de prolifération $\geq$ à 1+ 2$\sigma$ ($\sigma$ étant l'incertitude sur la numération cellulaire exprimée en %) signifie que le milieu contenant une PVP augmente la prolifération des cellules.

Par "mode batch" on entend un mode de culture en milieu fermé sans apport d'éléments nutritionnels additionnels, ni dispositif d'élimination des déchets toxiques qui se sont accumulés pendant la durée de la culture.

Par "mode Feed batch" on entend un mode de culture en milieu semi-fermé avec au moins un apport ponctuel d'éléments nutritionnels additionnels pendant la durée de la culture mais ce mode de culture ne comprend pas de dispositif d'élimination des déchets toxiques qui se sont accumulés.

Par "mode de culture en continu" on entend un mode de culture dans lequel du milieu de culture neuf est constamment apporté pour remplacer dans le même temps une quantité équivalente de milieu usagé qui est éliminé.

Par "mode de culture en continu sous perfusion" on entend un mode de culture en continu dans lequel il existe un dispositif supplémentaire qui retient les cellules.

Par "support d'adhésion" on entend une surface solide dont les composants chimiques externes en contact avec le milieu de culture permettent l'attachement des cellules adhérentes.

Par "bio-générateur" on entend une cuve de culture, généralement en inox, de volume supérieur à 2 Litres, comprenant un système d'agitation, un dispositif d'injection d'un courant gazeux de $CO_2$ et un dispositif d'oxygénation. Il est équipé de sondes mesurant les paramètres internes du bio-générateur comme le pH, l'oxygène dissous, la température, la pression de la cuve ou certains paramètres physico-chimiques de la culture (comme la consommation en glucose, de glutamine ou la production de lactates et d'ions ammoniums). Les sondes de pH, d'oxygène et de température sont reliées à un bio-processeur qui assure en permanence la régulation de ces paramètres.

Par "microporteurs" on entend des microbilles sphériques de 100 à 200$\mu$m de diamètre, poreuses ou non, dont la densité est très légèrement supérieure à celle du milieu de culture et recouvertes d'une matrice électrostatique adhésive. Les microporteurs sont maintenus en suspension par agitation mécanique.

Par "étape d'infection" on entend le temps de contact nécessaire pour des agents infectieux à cycle de reproduction intra-cellulaire strict tels que les virus, préparés sous la forme d'une suspension dans un milieu adapté, qualifié de milieu d'infection, puissent pénétrer dans la cellule.

Par "étape de propagation" on entend le temps nécessaire pour que des agents infectieux à cycle de reproduction intra-cellulaire strict, tels que les virus, puissent effectuer leur cycle de reproduction complet dans la cellule et qu'ils soient produits sous une forme mature. L'étape de propagation se fait dans un milieu de culture adapté, qualifié de milieu de propagation, qui peut être différent du milieu d'infection. Les formes matures de certains virus, comme le virus de la varicelle, restent intracellulaires ; la récolte du virus se fait alors au moyen de la lyse des cellules infectées. Dans d'autres cas, les formes matures sont extracellulaires et peuvent réinfecter d'autres cellules restées indemnes en réinitialisant de nouveaux cycles de reproduction intracellulaire. La récolte du virus peut se faire en une seule fois par prélèvement du milieu de propagation ou à intervalles réguliers, le milieu de propagation prélevé étant remplacé par du milieu neuf et ce, jusqu'à l'épuisement du cycle de reproduction intracellulaire par destruction complète de la couche cellulaire.

L'invention concerne donc l'utilisation d'une polyvinylpyrrolidone, préférentiellement non réticulée, d'un poids moléculaire moyen compris entre 20KD et 360 KD (PVP), et de façon préférée, une PVP d'un poids moléculaire moyen de 40KD, dans un milieu de culture dépourvu de sérum d'origine animale pour favoriser la multiplication de cellules animales ou humaines adhérentes soumises à un flux d'agitation du milieu. L'effet optimal sur la prolifération des cellules adhérentes comme par exemple sur des cellules d'embryons de poulets, sur des lignées murines comme la lignée 3T3, NTCT, WEHI, sur des lignées de hamster comme la lignée BHK ou CHO, sur des lignées canines comme la lignée MDCK ou les cellules primaires de rein de chien, sur des lignées porcines comme la lignée PK15, sur des lignées simiennes comme la lignée Vero, LLC-MK2, FRHL2 ou sur des lignées humaines comme la lignée MRC5, Hela, ECV ou A 431 ou les lignées de mélanomes comme la lignée A 375 est obtenu lorsqu'une PVP est utilisée à une concentration dans le milieu de culture comprise entre 0,01% et 2% (Poids/Volume). L'homme de l'art est en mesure d'adapter les concentrations d'utilisation de la PVP dans le milieu de culture en fonction du poids moléculaire moyen de celle ci. En effet, il est connu que plus le poids moléculaire moyen de la PVP est élevé, et plus la viscosité d'un milieu la contenant est importante. Une viscosité trop importante est gênante pour la croissance cellulaire. A titre indicatif, L'homme de l'art peut se référer à la valeur de la viscosité d'un milieu contenant une PVP de 40KD à une concentration de 0,1%, compatible avec une très bonne croissance cellulaire, pour déterminer les concentrations d'utilisation d'une PVP dans la gamme de poids moléculaire moyen préconisée pour réaliser l'invention.

Une PVP, d'un poids moléculaire moyen compris entre 20KD et 360 KD et de préférence de 40KD, utilisée à une concentration comprise entre 0,01% et 2% dans un milieu de culture sans sérum d'origine animal favorise aussi l'adhésion de, ces cellules ou de ces lignées cellulaires à leur support dans les heures qui suivent leur ensemencement et ceci en dépit d'une agitation du milieu de culture.

On a remarqué enfin qu'une PVP utilisée dans les mêmes conditions dans un milieu de culture sans sérum d'origine animale réduisait de façon sensible la mortalité cellulaire pendant toute la durée de la culture.

Une PVP selon l'invention, est généralement caractérisée par son poids moléculaire moyen mais peut être définie également au moyen de sa valeur K, qui prend en compte non seulement le poids moléculaire moyen d'une PVP mais également les variations de poids moléculaire de part et d'autre de la valeur moyenne. Pour le calcul de la valeur K on se réfère à l'équation telle que définie dans l'article Cryobiology, 8, 453-464 (1971): La valeur K est calculée à partir de la viscosité relative d'une solution à 1 % de PVP selon la formule

$$\text{Log } \eta \text{ rel}/C = 75 K_0^2 / (1 + 1.5\, K_0 C) + K_0$$

$K = 1000\, K_0$
C représente la concentration en grammes de PVP pour 100 ml de milieu.
$\eta$ rel est la viscosité de la solution comparée à celle du solvant.
Cette valeur K représente en définitive la viscosité intrinsèque d'une PVP qui à toutes fins utiles de l'invention devrait avoir une valeur comprise entre 20 et 100 pour une PVP ayant un poids moléculaire moyen compris entre 20KD et 360KD.

Le milieu de culture de base dans lequel est incorporé une PVP selon l'invention peut être du milieu MEM, MEM-$\alpha$, DMEM, RPMI, ISCOVE, Ham F12, HAM F10, M199, L15, 6M, NCTC109, du milieu de Fischer, de Waymouth, du milieu Néphros (milieu défini produit par Biowhittaker sous la référence n° 12-735Q), du milieu VPSFM (milieu défini fourni par la société Gibco Life Technologies et référencé sous le n°11002086), du milieu de Williams ou des mélanges de ces milieux de base. Ces milieux de base peuvent être enrichis en fonction des besoins des cellules, en facteurs nutritifs additionnels comme par exemple en sucres tels que le glucose, en acides aminés tels que la glutamine, d'un cocktail d'acides aminés non essentiels (fourni notamment par la société Gibco Life technologies) ou d'acides aminés essentiels, de peptides et plus particulièrement de peptides d'origine végétale, en acides ou sels d'acides tels que le pyruvate de sodium, sels d'EDTA, dérivés d'acide citrique ou plus généralement de dérivés d'acides impliqués dans le cycle de Krebs, en alcools comme l'éthanol, en amino-alcools comme l'éthanolamine, en vitamines comme les vitamines C et E, en agents anti-oxydants comme le gluthation ou le sélénium, en acides gras à chaînes saturées ou insaturées comme l'acide linoléique, l'acide arachidonique, l'acide oléique, l'acide stéarique ou l'acide palmitique, en lipides ou lipopeptides mais également en phospholipides tels que les lécithines et de façon préférentielle les lécithines d'origine végétale ou en précurseurs de ceux-ci. L'ajout d'une solution tampon à base d'HEPES ou de bicarbonates pourra s'avérer nécessaire pour certaines cultures cellulaires fragiles ou produisant de grandes quantités de $CO_2$, ou

éventuellement pour tamponner des milieux de culture fortement complémentés en acides. D'une façon générale on veillera à respecter un pH du milieu de culture compris entre 6 et 8, le plus souvent entre 7 et 8 et plus spécifiquement entre 7,2 et 7,5. On veillera autant que possible à respecter l'isotonicité du milieu de culture.

En fonction des besoins particuliers de certains types de cellules, on peut mettre avantageusement à profit l'objet de l'invention en ajoutant au milieu de culture des dérivés stéroïdiens comme le cortisol, des facteurs de croissance d'origine synthétique ou recombinante, tels que, par exemple, des cytokines, l'hormone de croissance (Growth hormone), les IGFs (insulin growth factor), l'EGF (epidermal growth factor), le FGF (Fibroblast growth factor), PDGF (platelet derivated growth factor), des facteurs d'attachement tels que le collagène recombinant. Cependant, ces facteurs ne sont pas absolument nécessaires pour réaliser l'invention. Même si préférentiellement on utilise une PVP dans un milieu de culture chimiquement défini, certaines cultures cellulaires, notamment les cultures primaires obtenues à partir d'explants d'organes peuvent nécessiter des compléments de culture à base d'extraits bactériens, de levures ou même de végétaux.

Il est bien entendu que l'énoncé des additifs n'est qu'indicatif mais ne saurait en aucun cas être considéré comme restrictif pour l'homme de métier voulant reproduire l'objet de l'invention.

Un autre aspect de l'invention concerne les différents procédés de culture utilisables pour assurer la croissance des cellules adhérentes à l'aide d'un milieu de culture conforme à l'invention. Les cultures de cellules adhérentes à l'aide d'un milieu de culture selon l'invention, peuvent être effectuées en "mode batch", "feed batch" ou en mode de "culture en continu" lorsque la croissance cellulaire atteint son palier dans un intervalle de 7 jours et lorsque la concentration cellulaire maximale n'excède pas $3 \cdot 10^6$ cellules /mL. Par contre, lorsque l'on souhaite obtenir des concentrations cellulaires maximales supérieures à $3 \cdot 10^6$ cellules /mL et/ou lorsque la durée de la culture est plus importante, le milieu de culture initial selon l'invention s'appauvrit trop en certains nutriments essentiels comme le glucose ou la glutamine pendant que s'accumulent les déchets toxiques issus du métabolisme cellulaire comme les lactates ou l'ammoniac. On utilise dans ce cas le mode "Feed-batch" ou le mode de "culture en continu". Le mode "Feed-batch" consiste à rajouter de façon ponctuelle certains des composants nutritifs de base contenu dans le milieu de culture initial selon l'invention qui ont été trop rapidement consommés par les cellules. Il s'agit généralement de rajouter de la Glutamine, ou des cocktails d'acides aminés, du glucose et parfois selon les cultures des composés lipidiques. Dans le mode de "culture en continu", on pallie aussi au problème de l'accumulation des déchets du fait que le milieu de culture selon l'invention est constamment renouvelé. L'homme de l'art, en suivant l'évolution des paramètres du métabolisme cellulaire, comme la mesure de la consommation du glucose ou de la glutamine, ou en suivant les taux d'ammoniac et de lactates dans le milieu culture est tout à fait apte à déterminer le mode de culture qui convient le mieux. Les dispositifs de culture permettant une agitation du milieu de culture comprennent les flacons roulants, les flacons de type "spinners", les bio-générateurs. Les supports de culture peuvent être constitués par les parois des flacons eux-mêmes sans être nécessairement traités au préalable par des agents adhésifs particuliers. On utilise également des microporteurs qui accroissent considérablement la surface disponible aux cellules adhérentes sans toutefois augmenter de façon importante le volume du milieu de culture nécessaire. Ce type de support avantageux requiert néanmoins une agitation permanente et souvent une bonne oxygénation du milieu de culture. On peut mettre à profit l'usage des microporteurs dans les flacons roulants, les flacons de type spinners ou les bio-générateurs. Les microporteurs peuvent être, selon les types cellulaires utilisés, constitués d'une matrice de dextran substituée par des groupements N, N diethylaminoéthyle (Cytodex 1 et Cytodex 2), ou éventuellement par des facteurs d'attachement d'origine synthétique ou recombinante comme la poly D lysine, le collagène recombinant ou le peptide RGD constitué de l'enchaînement d'arginine, de glycine et d'acide aspartique. Les microporteurs peuvent être aussi en polystyrène, en verre, en cellulose ou en polyacrylamide. Ils peuvent être utilisés à une concentration entre 1g/litre et 100g/litre de milieu de culture selon l'invention. Les microporteurs de type Cytodex1, sont utilisés de façon préférentielle à une concentration comprise entre 3g/litre et 30g/litre et de façon encore plus préférée à une concentration comprise entre 3g/litre et 15g/litre.

Lorsque les microporteurs de type Cytodex1 sont placés dans un biogénérateur à une concentration de 3g/litre, la concentration cellulaire maximale que l'on peut obtenir avec un milieu selon l'invention dépend de la taille des cellules et des conditions initiales d'ensemencement en cellules. En effet, pour qu'il y ait un recouvrement uniforme de la surface des microporteurs par les cellules, il faut que chaque microporteur soit colonisé par au moins une cellule lors de l'ensemencement. On a constaté que cette condition est remplie lorsque la concentration initiale d'ensemencement en cellules est comprise ente 15 et $50 \times 10^3$ cellules par $cm^2$ de surface en microporteurs. Dans ces conditions d'ensemencement, la concentration cellulaire maximale n'excède toutefois pas en général $3 \cdot 10^6$ cellules /mL en biogénérateur, lorsqu'on cultive des cellules de grande taille comme les cellules de la lignée Vero ou MRC5. Le palier de croissance est obtenu en général dans un délai de 7 jours après l'initiation de la culture si bien que le mode "batch", "feed batch" ou "de culture en continu" peut être utilisé. Par contre, lorsque les microporteurs de type Cytodex1 sont utilisés à une concentration de 15g/litre on obtient, dans les mêmes conditions optimales d'ensemencement, des densités cellulaires en cellules Vero ou MRC5 dépassant $5 \cdot 10^6$ cellules/mL en utilisant un milieu selon l'invention. Le mode "feed batch" ou le mode de "culture en continu" est alors utilisé pour pallier au déficit en nutriments essentiels et/ou au problème de l'accumulation des déchets toxiques. Quelque soit le mode utilisé, le milieu de culture est en permanence agité en utilisant par exemple une pale d'agitation mécanique, du type "delta" (brevet Fr: N°8018608). Lorsqu'on utilise un mode de "culture en continu sous perfusion", la pale d'agitation est placée de préférence au regard du dispositif qui retient les cellules dans le biogénérateur, ce dispositif étant par exemple une crépine. Le milieu, quelque soit le mode de culture utilisé, est aussi régulièrement oxygéné au moyen d'un tube creux plongeant, alimenté en oxygène. On peut aussi utiliser un procédé d'oxygénation du milieu selon l'invention situé à l'extérieur du biogénérateur, encore appelé oxygénateur. L'oxygénateur peut être constitué d'un système de fibres creuses dont la membrane en polysulfone, perméable à l'oxygène, permet le transfert d'oxygène dans le milieu de culture. Le milieu de culture du biogénérateur est également alimenté en $CO_2$ lorsque le PH s'abaisse. La vitesse d'agitation du milieu, de même que la teneur en oxygène du milieu selon l'invention sont des paramètres bien maîtrisables par l'homme de métier. Les index de prolifération obtenus en utilisant un procédé de culture selon l'invention et notamment le procédé de culture en biogénérateur avec des microporteurs à base de cytodex, dépassent souvent 1,5 et parfois sont proches de ceux observés avec des milieux de culture contenant du sérum d'origine animale utilisés pour la production industrielle de cellules.

L'objet de la présente invention sera maintenant mieux compris à la lecture des exemples qui suivent mais qui n'ont pas pour autant un quelconque caractère limitatif.

**Exemple 1: étude de la croissance d'une population de cellules Vero cultivées dans un bio-générateur à l'aide de milieux de culture chimiquement définis contenant une PVP.**

1A- Caractéristiques de la lignée Vero utilisée

La souche utilisée est issue de l'American Type Culture Collection (ATCC) référencée sous le code ATCC ccIVERO F 1415, passage 124. Une banque cellulaire de travail au 137<sup>ème</sup> passage de cette souche a été constituée. Les cellules Vero sont cultivées par passages successifs à partir de la banque de travail en milieu de culture additionné de sérum de veau jusqu'au 140<sup>ème</sup> passage. Elles subissent ensuite 2 à 3 passages dans l'un ou l'autre des deux milieux définis sans sérum suivant : le premier étant constitué à parties égales de milieu de Williams et de milieu Néphros, le second étant constitué à parties égales du milieu VPSFM et du milieu de Williams, ces passages supplémentaires étant réalisés de façon à disposer d'une quantité suffisante de cellules pour ensemencer un biogénérateur, un spinner ou des flacons roulants.

1B- Milieux de culture et réactifs utilisés

*Préparation des milieux de culture de base chimiquement définis*

Toutes les solutions préparées sont filtrées sur une unité Millex 0.22 $\mu$m (Millipore) afin de les stériliser.

- Milieu de Williams (voir annexe1)

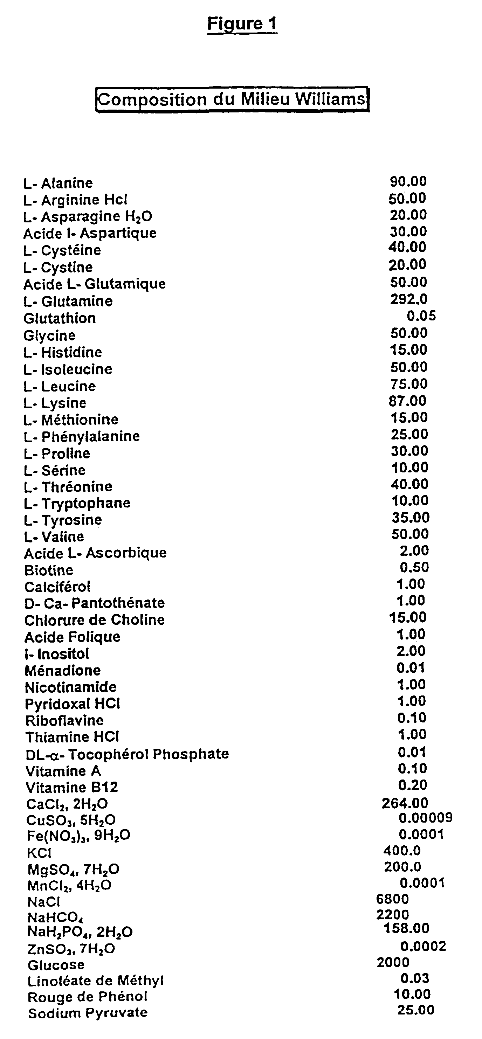

  - Mélange en poudre Williams (Gibco)     1 sachet dont les composants exprimés en grammes sont présentés dans la Figure 1
  - NaHCO$_3$ (Merck)     2,20 g
  - Gentamicine (Unicet)     50 mg
  - H$_2$O ultrafiltrée     QSP 1l

- Milieu Néphros (Biowhittaker- Ref n°12-735Q) : milieu prêt à l'emploi Ajout extemporané de :

  - Glutamine 200 mM     10 ml/l
  - Néomycine     1 ml/l

- Milieu VPSFM (Gibco Life Technologies- Ref n°11002086) prêt à l'emploi

*Formules et préparations des solutions et tampons utilisés en culture*

- PBS sans Ca$^{2+}$ ni Mg$^{2+}$

  - Solution 10 x c (Gibco)     10 ml

- H$_2$O ultrafiltrée      QSP 1l

- Tampon Citrate

  - NaCl      8,00 g
  - KCl      0,20 g
  - Na$_2$HPO4, 2H$_2$O      1,25g
  - KH$_2$PO4      0,20 g
  - Citrate trisodique, 2H$_2$O      7,40g
  - H$_2$O ultrafiltrée      QSP 1l

- Trypsine Sigma à 2.5%

  - Trypsine recristallisée 2 x (Sigma)      25,00 g
  - NaCl (Merck)      9,00 g
  - H$_2$O ultrafiltrée      QSP 1l

Le tampon citrate et la trypsine Sigma ont été filtrés sur cartouche Millipack 0.22 μm (Millipore). Le PBS a été filtré sur membrane 0.22 μm (Sartorius).

- Solution d'inhibiteur trypsique de Soja à 1 mg par litre

  - Inhibiteur trypsique de soja (Boehringer)      20 mg
  - Milieu E de Williams de base      20 ml

Cette solution peut être utilisée extemporanément ou conservée à - 20°C deux à trois semaines.

1C- Solution mère de PVP à 10% (Poids/Volume)

On dissout 50g de PVP 40KD (Sigma- ref 97H0571), ou 50g de PVP 360KD (Sigma -ref P5288) dans 500ml d'eau ultra filtrée suivie d'une filtration stérilisante sur unité Millex 0,22μm (Millipore). Les différentes solutions mère ainsi préparées sont conservées à +4°C et ajoutées en quantité adéquate au milieu de culture au moment de l'emploi en fonction des concentrations finales de PVP désirées.

1D- Description du bio-générateur et du Spinner

*Biogénérateur de 3 litres*

Il est constitué d'une cuve en inox à fond rond munie d'un système d'agitation constitué d'une pale delta. Il est équipé de sondes pH, pression en O$_2$, de température et relié à un pupitre BP 2.10 IM (PMC) qui assure en continu la régulation des différents paramètres au moyen d'injection de CO$_2$, celle de la teneur en oxygène dissout par un balayage d'air en surface et d'une injection d'oxygène en profondeur. La température est régulée à l'aide d'un cryostat RM6 Lauda qui maintient la culture à 37°C grâce à une double enveloppe dans laquelle l'eau du cryostat passe en circuit fermé.

Pour certains essais de culture en continu sous perfusion, d'autres systèmes ont été utilisés. Un système de perfusion a été mis en place (Applikon Biosep ADI 1015) faisant intervenir une chambre de résonance à ultrasons permettant d'éliminer du milieu usagé tout en relargant les microporteurs dans le biogénérateur. Ce système nécessite l'utilisation de 2 pompes, une pompe de recirculation (Masterflex console drive, model 7518-02) et une pompe à deux têtes Minipulse 2 (Gilson), pour l'alimentation et le rejet du biogénérateur. Le tout est géré par une unité Applikon Biosep ADI 1015 régulant la puissance et la fréquence du système et contrôlant la pompe de recirculation et la chambre de résonance.

*Spinner de 250 ml (Integra Biosciences)*

Ils sont en verre avec deux bouchons a chaque extrémité. Le système d'agitation nécessite un agitateur magnétique. Le système dans le spinner est constitué de 2 tiges lisses et rondes contenant un barreau aimanté. La rotation est assurée par un plateau magnétique Cellspin (Integra Biosciences). La mesure des paramètres se fait manuellement.

*pH mètre*

Le pH des milieux après préparation et en cours de culture est contrôlé avec un pH mètre (PHM 220) Minissis 5000 Tacussel électronique.

1E- Culture des cellules Vero en Bio-générateur de 3 litres (ou en Spinner de 250ml) sur microporteurs de type Cytodex1

Des billes de Cytodex1 (Pharmacia) sont hydratées pendant 24 heures à raison de 1g de billes pour 50ml environ de tampon phosphate (0,1M, pH= 7,4) suivi de 5 à 10 lavages dans le même tampon jusqu'à ce que le pH du tampon redevienne normal. Les billes sont ensuite stérilisées pendant 1 heure à 121°C sous 1,5-2 bars. Juste avant utilisation les microporteurs sont rincés 2 fois avec le milieu devant être utilisé pour la culture cellulaire avant d'être placés à raison de 3g/litre de milieu dans un Spinner ou un Biogénérateur en vue d'une production de cellules faiblement concentrée (« culture basse densité ») ou à raison de 15g/litre de milieu dans un Biogénérateur en vue d'une production de cellules fortement concentrée (« culture haute densité »). Les cellules Vero prêtes à l'emploi sont ensemencées à raison de 30 x 10$^3$ cellules par cm$^2$ de surface cultivable. Le volume final de milieu correspond au volume recommandé pour le système de culture utilisé (3 litres pour un Bio-générateur, 200 ml pour un Spinner de 250 ml). Selon les cas, le milieu de culture utilisé est constitué soit du mélange à parties égales du milieu Nephros et du milieu de Williams auquel est ajouté une PVP 40KD à la concentration finale de 0,1%, soit du mélange à parties égales du milieu VPSFM et du milieu de Williams auquel est ajouté une PVP 40KD à la concentration finale de 0,1%, soit du mélange à parties égales du milieu VPSFM et du milieu de Williams auquel est ajouté du sérum de veau donneur à la concentration finale de 4%, ou soit enfin un milieu de culture de référence optimisé pour la production industrielle des cellules Vero constitué d'une base ISCOVE auquel est ajouté du sérum de veau donneur à la concentration finale de 4%. L'agitation mécanique du milieu est fixée à environ 30 tours/minutes. Pour la « culture basse densité », l'aération de surface est faite par un mélange d'air à 5% de CO2 tandis que l'oxygénation est faite en profondeur de façon à avoir au minimum une quantité d'oxygène dissoute dans le milieu équivalente à au moins 10% de la saturation maximale en oxygène du milieu. La Culture peut être maintenue dans ces conditions pendant 5 à 7 jours sans changement ou apport de milieu que ce soit en Spinner ou en Bio-générateur. Pour la « culture haute densité » réalisée en Bio générateur, le milieu de culture est renouvelé à partir du deuxième jour de culture jusqu'au septième jour de culture à raison de deux volumes de milieu de culture par 24 heures (culture selon un mode de culture en continu sous perfusion). L'agitation mécanique de la pale est fixée à environ 15 tours/minutes. La quantité d'oxygène dissoute dans le milieu est équivalente à 25% de la saturation maximale en oxygène du milieu.

1F- Etude des paramètres métaboliques de la culture

Le suivi des nutriments essentiels et métabolites est assuré par un système d'analyse Nova Biomedical (Bio Profile 200) mesurant le pH, l'osmolarité du milieu, et les taux de glutamine, de glucose, de glutamate, de lactate, d'ammoniaque et de certains ions tels que le Ca$^{2+}$, le K$^+$ et le Na$^+$. Cet analyseur nécessite un échantillon de surnageant de milieu de 500 µl.
Les composés les plus intéressants à suivre sont d'une part le glucose et la glutamine, sources d'énergie de la cellule et d'autre part leurs produits de dégradation que sont le lactate et l'ammoniaque. La mesure de ces éléments permet de contrôler l'état cellulaire de la culture.

1G- Etude de la croissance cellulaire

La croissance cellulaire est évaluée quotidiennement au moyen du nombre de cellules fixées sur les billes par la technique de coloration des noyaux selon la méthode de Sanford K. et al (J. Nat. Cancer Inst., vol. 11, 773-795 (1951)) et de Van Wezel A. (Microcarrier culture of animal cells. Tissue culture: methods and applications, Kruse P.F. et Patterson M.K., Eds, Academic Press, New York, 372-377 (1973))
Dans un tube de 15 ml, on introduit 5 ml d'un prélèvement homogène d'une culture en biogénérateur ou spinner. Après décantation des billes et élimination du surnageant, les billes colonisées par les cellules sont rincées 2 fois avec un tampon phosphate. Sur le culot du dernier rinçage on rajoute 5ml d'une solution de cristal violet à 0,1%. Au bout d'une incubation de 45 minutes à 37°C, l'acide citrique contenu dans le cristal violet a libéré et coloré tous les noyaux des cellules. La suspension de noyaux est alors homogénéisée par agitation puis les noyaux énumérés sur lame de Fuchs-Rosenthal . Le nombre de noyaux reflète le nombre de cellules fixées sur les microporteurs qui est rapporté au nombre de cellules par ml de milieu. L'incertitude sur la mesure est de +/-10%. Les résultats des numérations cellulaires exprimées en nombre de cellules/ml sont indiqués dans le tableau ci-dessous.

Tableau 1

| | N/W | N/W+PVP | VPSFM/W | VPSFM/W +PVP | VPSFM/W +SVD | ISCOVE +SVD |
|---|---|---|---|---|---|---|
| J0 | 675000* | 675000 | 405000 | 405000 | 405000 | 405000 |
| J1 | 307810 | 1562500 | 533000 | 781250 | 881000 | 573000 |
| J2 | 646875 | 2375000 | 997000 | ND | 2210000 | 1430000 |
| J3 | 868750 | 3250000 | 1390000 | 2006250 | 2230000 | 1740000 |
| J4 | 1765625 | 2906250 | 1670000 | 2162500 | 2590000 | 1950000 |
| J5 | 1187500 | 2468750 | 1800000 | 1993750 | ND | 2330000 |
| J6 | 1023440 | 2046875 | 1610000 | 1887500 | 1990000 | 1920000 |

Légendes:

J0, J1, J2, J3, J4, J5, J6 représentent les temps de prélèvements pour numération des cellules, le temps J0 correspondant à la concentration cellulaire dans le milieu après ensemencement des cellules dans le Bio-générateur dans le cas d'une culture « basse densité », J1 le temps après 24 heures de culture, J2 le temps 48 heures, J3 le temps 72 heures, J4 le temps 96 heures, J5 le temps 120 heures, J6 le temps 144 heures.

*: nombre de cellules/ml N/W: Milieu de Néphros (Biowhittaker)Williams N/W+PVP: Milieu de Néphros (Biowhittaker)/Williams additionné d'une PVP 40KD à 0,1% VPSFM/W: Milieu VPSFM (Life Technology)/ Williams VPSFM/W+PVP: Milieu VPSFM (Life Technology)/ Williams additionné d'une PVP 40KD à 0,1% VPSFM+SVD: Milieu VPSFM/Williams additionné de 4% de sérum de veau donneur ISCOVE+SVD: Milieu Iscove additionné de 4% de sérum de veau donneur.

ND: Non déterminé

Conclusions:

Bien que les conditions opératoires et les concentrations cellulaires d'ensemencement soient identiques le milieu N/W+PVP augmente la prolifération des cellules Vero et permet d'atteindre des concentrations cellulaires maximales significativement plus importantes que celles obtenues avec le milieu N/W seul (3 25000 à J3 versus 1 765625 à J4). L'index de prolifération constaté avec le milieu N/W+PVP est de 1,84 et est très nettement au dessus de 1,2 (1+2X0,1 (10% étant l'incertitude sur la mesure précisée dans les conditions expérimentales). On observe également la même tendance avec le milieu VPSFM/W dans lequel le PVP accélère également la croissance cellulaire (l' index de prolifération est de 1,201).

L'action de la PVP sur la croissance cellulaire des cellules adhérentes n'est donc pas dépendante du milieu utilisé. De plus, la PVP 40 procure des résultats substantiellement similaires à ceux obtenus avec le sérum de veau puisque les concentrations cellulaires mesurées durant les jours 1 à 6 sont sensiblement du même ordre. Les résultats obtenus avec une PVP 360KD à la concentration finale de 0,5% dans le milieu de culture sont similaires à ceux obtenus avec une PVP 40. Par contre un milieu contenant une PVP 10KD à la concentration finale de 0,1% dans le milieu de culture n'a pas d'effet positif sur la croissance cellulaire.

Nous avons également étudié la croissance des cellules Vero en Biogénérateur, placées dans des conditions de culture dite de « haute densité » et dans lesquelles le milieu de culture est un mélange à parties égales de VPSFM et de milieu Williams auquel a été ajouté une PVP 40KD à la concentration finale de 0,1%. Les valeurs des concentrations cellulaires obtenues entre J0 et J6 sont répertoriées dans le tableau n°2 et exprimées en millions de cellules/ml.

Tableau 2

| | J0 | J1 | J2 | J3 | J4 | J5 | J6 |
|---|---|---|---|---|---|---|---|
| VPSFM/W+PVP | 2,05 | 2,00 | 2,85 | 4,05 | 5,85 | 5,95 | 5,80 |

Ces résultats montrent que la concentration cellulaire a triplé entre J0 et J6. Un milieu exempt de sérum d'origine animale et contenant une PVP comme substitut du sérum peut donc être utilisé pour des cultures dites de « haute densité » en biogénérateur.

## Exemple 2: Etude de l'action du PVP sur la mortalité cellulaire

Les conditions opératoires de l'étude sont identiques à celles développées aux paragraphes 1A à 1F.

La mortalité cellulaire est évaluée quotidiennement au moyen du nombre de cellules mortes ou de la mesure de la LDH (lactate deshydrogénase) retrouvée dans le milieu de culture. La mortalité cellulaire est évaluée au moyen d'une coloration au bleu trypan. A partir d'un prélèvement quotidien de 1ml de la culture cellulaire on rajoute 1ml de bleu trypan à 10%. Les cellules mortes qui prennent le colorant sont comptées à l'aide d'une cellule de Fuchs-Rosenthal. L'incertitude sur la mesure est de +/- 10%. Les résultats de mortalité cellulaire, exprimés en nombre de cellules/ml, sont indiqués dans le tableau 3.

L'évaluation de la mortalité cellulaire peut être également faite au moyen de la mesure de la lactate deshydrogénase dans le surnageant de culture. En effet, il a été montré qu'une évaluation de la mortalité cellulaire au cours de la fermentation dans les bio-réacteurs peut être réalisée par la mesure de la LDH relarguée dans le surnageant de culture. Un prélèvement quotidien de 0,5 ml est centrifugé à 800 tours/minute pendant 5 minutes pour éliminer les cellules. Il est alors incubé avec le mélange de réactifs du kit (Cytoxicity Détection kit (LDH), Boehringer Mannheim, Cat. No 1644 793) selon la procédure décrite dans le coffret. Une augmentation de la mortalité cellulaire ou des lésions membranaires entraîne une augmentation de l'activité LDH se traduisant par une augmentation de la densité optique (ou de coloration) dans l'essai utilisé. Le dosage est réalisé en plaques 96 puits (Evergreen Scientific) à fond plat avec un couvercle de polystyrène non traité. Dans 2 puits on dépose 200µl du surnageant à doser ou des dilutions de celui-ci. On rajoute 100µl de réactif du coffret par puits. Après une incubation de 10 minutes à 37°C dans une étuve sèche on bloque la réaction enzymatique par 50µl d'HCl 1N. La densité optique (DO) est mesurée à 490nm au moyen d'un lecteur de microplaques (Molecular devices Emax). La précision de la mesure est +/- 10%. Plus la DO est élevée et plus la mortalité cellulaire est importante. Les résultats comparatifs entre les différents milieux testés, exprimés en D.O. de l'échantillon testé, sont regroupés dans le tableau 4.

Tableau 3

| Prélèvement | N/W+PVP | VPSFM/W | VPSFM/W +PVP | ISCOVE +SVF |
|---|---|---|---|---|
| J0 | N.D. | N.D. | N.D. | N.D. |
| J1 | < | 19063 | < | 51250 |
| J2 | < | 23125 | < | 48440 |
| J3 | < | 35313 | < | 52810 |
| J4 | < | 16875 | < | 42500 |
| J5 | < | 61560 | < | 74690 |
| N.D. : non déterminé<br><: inférieur à 5000 cellules /ml | | | | |

Tableau 4

| Prélèvement | N/W+PVP | VPSFM/W | VPSFM/W +PVP | ISCOVE +SVF |
|---|---|---|---|---|
| J0 | N.D. | N.D. | N.D. | N.D. |
| J1 | 0,027 | 0,110 | 0,028 | 0,055 |
| J2 | 0,028 | 0,230 | N.D. | 0,073 |
| J3 | 0,065 | 0,390 | 0,066 | 0,153 |
| J4 | 0,112 | 0,510 | 0,115 | 0,155 |
| J5 | 0,140 | 0,600 | 0,226 | 0,268 |
| N.D. : non déterminé | | | | |

Conclusions:

Tandis que les résultats obtenus avec des milieux sans sérum ou avec sérum montrent une mortalité cellulaire fluctuant entre $10^4$ et $10^5$ cellules mortes/ml en l'absence de PVP, ceux obtenus avec des milieux définis additionnés de PVP montrent une diminution très significative de la mortalité cellulaire.

Les résultats de mesure de l'activité LDH dans les surnageants de culture montrent également une activité plus faible dans les surnageants de culture provenant de milieux contenant une PVP que ceux dépourvus de PVP. On peut noter également que l'activité LDH est plus élevée dans les surnageants de culture issus d'un milieu à base de sérum que celle présente dans des milieux contenant une PVP. Ces résultats corrèlent donc avec ceux de la mortalité cellulaire. On peut donc conclure que les milieux de culture contenant une PVP assure une meilleure intégrité cellulaire en diminuant très sensiblement la mortalité cellulaire, cette action étant plus importante que celle observée avec un milieu contenant du sérum de veau.

**Exemple 3: Étude de l'action du PVP sur l'adhésion des cellules à leur support**

Les conditions opératoires de l'étude sont identiques à celles développées aux paragraphes 1A à 1F.

Pour évaluer l'effet d'une PVP sur l'adhésion des cellules, on détermine le nombre de cellules qui se sont fixées sur les microporteurs 4 heures après l'ensemencement du Bio-générateur ou du Spinner selon la technique développée au paragraphe 1G. On considère qu'au bout de 4 heures, les cellules Vero sont toujours dans la même phase de leur cycle cellulaire, ce qui signifie que le nombre de noyaux énumérés correspond au nombre de cellules attachées. L'incertitude sur la mesure est de +/- 10%. Les résultats exprimés en nombre de cellules/ml sont regroupés dans le tableau ci-dessous.

Tableau 5

| Prélèvement | N/W | N/W+PVP | VPSFM/W | VPSFM/W +PVP |
|---|---|---|---|---|
| 0 | 675000* | 675000 | 405000 | 405000 |
| 4h | 453125 | 787500 | 394000 | 439250 |

Conclusions:

Ces résultats montrent clairement que la concentration cellulaire est plus importante au bout de 4 heures lorsque les milieux dépourvus en sérum contiennent une PVP.

**Exemple 4: Rôle d'une PVP sur la croissance, l'adhésion et la mortalité des cellules Vero cultivées en flacons roulants.**

Les cellules Vero telles que décrites dans le paragraphe 1A sont ensemencées dans des flacons roulants de 300ml (Ref: Falcon 3007) à raison de 30000 à 50000 cellules par cm$^2$ de support. Différents milieux de culture définis et non définis exempts de sérum d'origine animale mais contenant une PVP 40KD à 0,1% ont été testés dont ceux décrits dans le paragraphe 1A. Une PVP de 100KD à 0,5% ainsi qu'une PVP de 360KD à 0,05% ont été également testés en utilisant les mêmes milieux de culture.

Les cellules Vero en suspension dans les différents milieux de culture testés sont alors introduites dans des flacons roulants placés ensuite sur des rouleaux dont la vitesse de rotation se situe entre 0.1 tours/mn et 0.5 tours/mn. Quatre heures après l'ensemencement des flacons, puis quotidiennement pendant 6 jours, on réalise sur ces flacons roulants une étude de la croissance et de l'adhésion des cellules aux parois du flacon après avoir enlevé le milieu de culture, lavé les flacons avec un tampon phosphate et décollé les cellules avec de la trypsine à 2,5 % (Sigma) diluée au 1/5000 dans un tampon citrate. La numération des cellules se fait à l'aide du bleu trypan. L'étude de la mortalité des cellules a été suivie également sur une période de 5 jours à partir de la mesure de la LDH dans le surnageant de culture. Les résultats de ces études corroborent ceux décrits dans les exemples précédents et indiquent qu'on peut utiliser une PVP dans une large gamme de poids moléculaire moyen mais néanmoins supérieur à 10 KD et dans une large gamme de concentration dans un milieu de culture dépourvu en sérum d'origine animale pour favoriser la prolifération et l'attachement de cellules adhérentes et diminuer très sensiblement la mortalité cellulaire.

**Exemple 5: Rôle d'une PVP sur la croissance, l'adhésion et la mortalité des cellules MRC5 cultivées en flacons roulants.**

Les cellules MRC5 (Ref: NIBSC (pdl8)) utilisées entre le 20$^{ème}$ et 50$^{ème}$ passage sont ensemencées dans des flacons roulants de 300ml (Ref: Falcon 3007) à raison de 50000 cellules par cm$^2$ de support. Différents milieux de culture définis exempts de sérum d'origine animale mais contenant une PVP 40KD à 0,1% ont été testés dont ceux décrits dans le paragraphe 1A. Une PVP de 100KD à 0,1% ainsi qu'une PVP de 360KD à 0,05% ont été également testés en utilisant les mêmes milieux de culture.

Les flacons roulants contenant les cellules MRC5 ainsi que les différents milieux de culture mentionnés sont ensuite placés sur des rouleaux dont la vitesse de rotation se situe entre 0.1 tours/mn et 0.5 tours/mn. 4 heures après l'ensemencement des flacons, puis quotidiennement pendant 4 jours, on réalise sur ces flacons roulants une étude de la croissance et de l'adhésion des cellules aux parois du flacon après avoir enlevé le milieu de culture, lavé les flacons avec un tampon phosphate et décollé les cellules avec de la trypsine à 2,5 % (Sigma) diluée au 1/5000 dans un tampon citrate. La numération des cellules se fait à l'aide du bleu trypan. L'étude de la mortalité des cellules a été suivie également sur une période de 4 jours à partir de la mesure de la LDH dans le surnageant de culture. Les résultats de ces études correspondent à ceux obtenus avec les cellules Vero et montrent que les effets d'une PVP sur la croissance, la mortalité et l'adhésion cellulaire ne sont pas limités à un seul type cellulaire.

## Revendications

1. Procédé de production dans un milieu de culture dépourvu en sérum d'origine animale, de cellules animales ou humaines non recombinantes adhérentes comprenant:

   (i) une première étape dans laquelle on ensemence dans un dispositif de culture comprenant un support d'adhésion, un milieu de culture dépourvu en sérum d'origine animale et contenant une polyvinylpyrrolidone d'un poids moléculaire moyen compris entre 20 kDa et 360 kDa avec une suspension de cellules animales ou humaines non recombinantes ;
   (ii) une deuxième étape dans laquelle les cellules se multiplient sous agitation dans le même milieu de culture; et
   (iii) une troisième étape dans laquelle on récolte les cellules lorsqu'elles ont atteint un pallier de croissance.

2. Procédé selon la revendication 1, dans lequel le milieu de culture a une composition chimiquement définie.

3. Procédé selon la revendication 1 ou 2, dans lequel la polyvinylpyrrolidone a un poids moléculaire moyen de 40 kDa.

4. Procédé selon l'une des revendications 1 à 3, dans lequel le pourcentage de polyvinylpyrrolidone dans le milieu de culture est compris entre 0,01% et 2%.

5. Procédé selon l'une des revendications 1 à 4, dans lequel le pourcentage de polyvinylpyrrolidone dans le milieu de culture est de 0,1%.

6. Procédé selon l'une des revendications 1 à 5, dans lequel le support d'adhésion est constitué par des microporteurs.

7. Procédé selon la revendication 6, dans lequel les microporteurs sont constitués par une matrice de dextran substituée par des groupements N, N diethylaminoéthyle.

8. Procédé selon l'une des revendications 1 à 7, dans lequel le dispositif de culture est un biogénérateur.

9. Procédé selon l'une des revendications 1 à 8, dans lequel le mode de culture est le mode batch, feed batch ou par perfusion continue.

10. Procédé selon l'une des revendications 1 à 9, dans lequel les cellules sont les cellules de la lignée Vero ou MRC5.

## Patentansprüche

1. Verfahren zur Erzeugung von adhärenten, nicht rekombinanten tierischen oder menschlichen Zellen in einem Kulturmedium, das frei von Serum tierischen Ursprungs ist, umfassend:

   (i) einen ersten Schritt, in dem man in einer Kulturvorrichtung, die ein Adhäsionssubstrat umfasst, ein Kulturmedium, das frei von Serum tierischen Ursprungs ist und ein Polyvinylpyrrolidon mit einem mittleren Molekulargewicht zwischen 20 kDa und 360 kDa enthält, mit einer Suspension nicht rekombinanter tierischer oder menschlicher Zellen beimpft,
   (ii) einen zweiten Schritt, in dem sich die Zellen unter Rühren in demselben Kulturmedium vermehren, und

(iii) einen dritten Schritt, in dem man die Zellen erntet, wenn sie ein Wachstumsplateau erreicht haben.

2. Verfahren gemäß Anspruch 1, worin das Kulturmedium eine chemisch definierte Zusammensetzung hat.

3. Verfahren gemäß Anspruch 1 oder 2, worin das Polyvinylpyrrolidon ein mittleres Molekulargewicht von 40 kDa hat.

4. Verfahren gemäß einem der Ansprüche 1 bis 3, worin der Prozentsatz an Polyvinylpyrrolidon in dem Kulturmedium zwischen 0,01 % und 2 % liegt.

5. Verfahren gemäß einem der Ansprüche 1 bis 4, worin der Prozentsatz an Polyvinylpyrrolidon in dem Kulturmedium 0,1 % beträgt.

6. Verfahren gemäß einem der Ansprüche 1 bis 5, worin das Adhäsionssubstrat aus Mikrocarriern besteht.

7. Verfahren gemäß Anspruch 6, worin die Mikrocarrier aus einer Dextranmatrix, die mit N,N-Diethylaminoethylgruppen substituiert ist, besteht.

8. Verfahren gemäß einem der Ansprüche 1 bis 7, worin die Kulturvorrichtung ein Biogenerator ist.

9. Verfahren gemäß einem der Ansprüche 1 bis 8, worin die Art der Kultur der Batch- oder Feed-Batch-Betrieb ist oder durch kontinuierliche Perfusion erfolgt.

10. Verfahren gemäß einem der Ansprüche 1 bis 9, worin die Zellen Zellen der Vero- oder MRC5-Linie sind.

**Claims**

1. Process for producing in a culture medium free of serum of animal origin, adherent nonrecombinant animal or human cells, comprising:

(i) a first step in which a culture medium free of serum of animal origin and containing a polyvinylpyrrolidone with an average molecular weight of between 20 kDa and 360 kDa is inoculated with a suspension of nonrecombinant animal or human cells in a culturing device comprising an adhesion support;
(ii) a second step in which the cells multiply under agitation in the same culture medium; and
(iii) a third step in which the cells are harvested when they have reached a growth stage.

2. Process according to Claim 1, in which the culture medium has a chemically defined composition.

3. Process according to Claim 1 or 2, in which the polyvinylpyrrolidone has an average molecular weight of 40 kDa.

4. Process according to one of Claims 1 to 3, in which the percentage of polyvinylpyrrolidone in the culture medium is between 0.01% and 2%.

5. Process according to one of Claims 1 to 4, in which the percentage of polyvinylpyrrolidone in the culture medium is 0.1%.

6. Process according to one of Claims 1 to 5, in which the adhesion support consists of micro carriers.

7. Process according to Claim 6, in which the micro carriers consist of a dextran matrix substituted with N,N-diethylaminoethyl groups.

8. Process according to one of Claims 1 to 7, in which the culturing device is a biogenerator.

9. Process according to one of Claims 1 to 8, in which the culture mode is the batch or feed batch mode or the continuous perfusion mode.

10. Process according to one of Claims 1 to 9, in which the cells are cells of the Vero or MRC5 line.

## Figure 1

**Composition du Milieu Williams**

| | |
|---|---|
| L- Alanine | 90.00 |
| L- Arginine Hcl | 50.00 |
| L- Asparagine $H_2O$ | 20.00 |
| Acide l- Aspartique | 30.00 |
| L- Cystéine | 40.00 |
| L- Cystine | 20.00 |
| Acide L- Glutamique | 50.00 |
| L- Glutamine | 292.0 |
| Glutathion | 0.05 |
| Glycine | 50.00 |
| L- Histidine | 15.00 |
| L- Isoleucine | 50.00 |
| L- Leucine | 75.00 |
| L- Lysine | 87.00 |
| L- Méthionine | 15.00 |
| L- Phénylalanine | 25.00 |
| L- Proline | 30.00 |
| L- Sérine | 10.00 |
| L- Thréonine | 40.00 |
| L- Tryptophane | 10.00 |
| L- Tyrosine | 35.00 |
| L- Valine | 50.00 |
| Acide L- Ascorbique | 2.00 |
| Biotine | 0.50 |
| Calciférol | 1.00 |
| D- Ca- Pantothénate | 1.00 |
| Chlorure de Choline | 15.00 |
| Acide Folique | 1.00 |
| l- Inositol | 2.00 |
| Ménadione | 0.01 |
| Nicotinamide | 1.00 |
| Pyridoxal HCl | 1.00 |
| Riboflavine | 0.10 |
| Thiamine HCl | 1.00 |
| DL-α- Tocophérol Phosphate | 0.01 |
| Vitamine A | 0.10 |
| Vitamine B12 | 0.20 |
| $CaCl_2, 2H_2O$ | 264.00 |
| $CuSO_3, 5H_2O$ | 0.00009 |
| $Fe(NO_3)_3, 9H_2O$ | 0.0001 |
| KCl | 400.0 |
| $MgSO_4, 7H_2O$ | 200.0 |
| $MnCl_2, 4H_2O$ | 0.0001 |
| NaCl | 6800 |
| $NaHCO_4$ | 2200 |
| $NaH_2PO_4, 2H_2O$ | 158.00 |
| $ZnSO_3, 7H_2O$ | 0.0002 |
| Glucose | 2000 |
| Linoléate de Méthyl | 0.03 |
| Rouge de Phénol | 10.00 |
| Sodium Pyruvate | 25.00 |